# EUROPEAN PATENT SPECIFICATION

(11) **EP 1 414 419 B1**
(45) Date of publication and mention of the grant of the patent: **29.11.2006**
(21) Application number: 02751341.5
(22) Date of filing: 26.07.2002
(51) Int. Cl.: A61K 9/52, A61K 31/496

(54) **PHARMACEUTICAL FORMULATIONS COMPRISING A CORTISOL SYNTHESIS INHIBITOR**
PHARMAZEUTISCHE ZUBEREITUNGEN MIT EINEM CORTISOL SYNTHESE INHIBITOR
PREPARATIONS PHARMACEUTIQUES CONTENANT UN INHIBITEUR DE LA SYNTHESE DE CORTISOL

(30) Priority: 26.07.2001 GB 0118300
(43) Date of publication of application: 06.05.2004
(73) Proprietor: Cortendo AB, 421 30 Västra Frölunda (SE)
(72) Inventor: LJUSBERG, Barbro, Helena, 236 33 Höllviken (SE); HYLEN, Susanne, Veronica, 216 23 Malmö (SE); FYGE, Asa, Helen, 231 95 Trelleborg (SE)
(74) Representative: Gardner, Rebecca Katherine
(86) International application number: PCT/GB2002/003444
(87) International publication number: WO 2003/011258

(56) References cited:
- WO-A-00/00179
- WO-A-01/01971
- WO-A-01/52833
- US-A- 4 369 172

## Description

The present invention relates to compositions and formulations comprising ketoconazole and their use in therapy. More particularly, the invention relates to the use of such compositions in the prevention or treatment of metabolic syndrome and non-insulin-dependent diabetes mellitus (NIDDM), referred to herein as diabetes mellitus type II.

Often a person with abnormal glucose tolerance, insulin resistance or diabetes mellitus type II will also have one or more risk factors for cardiovascular disease, stroke or other arteriosclerotic disease such as obesity, especially abdominal obesity, hypertension, dyslipidemia and microalbuminuria. This clustering has been labelled variously as metabolic syndrome, Syndrome X or Insulin Resistance Syndrome. An attempt to more adequately describe metabolic syndrome was recently done (Diabet. Med. 1998, 15, 539-553) and a working definition for the clinical recognition of the condition was suggested to include the following characteristics: glucose intolerance, abnormal glucose tolerance or diabetes mellitus and/or insulin resistance together with two or more of the components: (1) Impaired glucose regulation or diabetes (2) Insulin resistance (3) Raised arterial blood pressure (4) Raised plasma triglycerides and/or low HDL-cholesterol (5) Central obesity and/or a body mass index of more than 30 kg/m² (6) Microalbuminuria.

There is still no generally accepted mechanism or mechanisms explaining the development of Metabolic Syndrome. Nevertheless, given the fact that one of the most important, and probably one of the earliest disturbance in the Metabolic Syndrome, is the accumulation of visceral (intra-abdominal) fat, regulators for this accumulation must be considered as important. A major regulatory mechanism for this accumulation seems to be cortisol, which is known to stimulate the storage of fat in this depot. This hormone is also known to increase the metabolic activity of visceral adipose tissue, which in turn is expected to result in an increase of free fatty acid exposure to the liver. When these free fatty acids are oxidized in the liver this results in a weakened biological binding of insulin to its receptors on hepatocytes, in turn leading to peripheral hyperinsulinemia and a less effective metabolic regulation by the liver resulting in hyperglycemia, dyslipidemia, insulin resistance and diabetes mellitus type II. These mechanisms (mainly insulin resistance) also lead to hypertension. Thus, one central pathogenic mechanism for the development of the Metabolic Syndrome could be an increased secretion and/or biological activity of cortisol.

Metabolic syndrome is characterised by a breakdown in the negative feedback mechanism which operates along the hypothalamic-pituitary-adrenal gland axis and results in increased cortisol secretion. It is therefore thought desirable to reduce cortisol activity in the body through exogenous means which can go some way to mimicking regulation by negative feedback observed in healthy people.

Those patients with metabolic syndrome are at very high risk of developing diabetes and therefore vigorous early management of metabolic syndrome may have a significant impact on the prevention of diabetes. All of the physiological imbalances associated with metabolic syndrome are risk factors for cardiovascular disease and thus effective early management of metabolic syndrome may also have a significant impact on the prevention of cardiovascular disease, stroke and other arteriosclerotic diseases.

Commercially available medical treatment for metabolic syndrome is generally lacking. Currently, most widely used therapies include diet and exercise (weight reduction therapy). Other therapies that have been suggested for the treatment are disclosed in US 5,756,513, US 5,849,740, and WO 98/36770, using a dopamine agonist, ketoconazole, or a combination of a cortisol synthesis inhibitor (such as ketoconazole) and human growth hormone, respectively. The use of ketoconazole as described in US 5,849,740 is based on an already marketed oral immediate release dosage form, Nizoral^{®} (Fungoral^{®}), originally developed by Janssen Pharmaceutics (Belgium) for the treatment of fungal infection and containing a racemic mixture of the two cis isomers.

Thus, the utility of a cortisol synthesis inhibitor in the treatment of metabolic syndrome and related conditions has been shown. However, the normal 24 hour plasma profile of cortisol shows a circadian variation with a shallow minimum during the day time (with the exception of short peaks of increased secretion) until around midnight followed by a rather steep increase to a broad maximum during the night, followed by a sharp fall during early morning lasting until around eight in the morning. It is now realised that administration of an immediate release dosage form of active agent, even around 10p.m. as proposed in US 5,849,740, may not provide an effective enough counter to the rising plasma cortisol levels which occur in the early hours of the morning.

Metabolic syndrome and diabetes mellitus type II are typical 'Western' diseases and effective treatment would benefit a very large number of people. Many sufferers are sophisticated health care users and a therapeutic method which was both effective and convenient to administer and receive would represent a significant advantage. A single daily dose is preferred and this should preferably be given at a time of day when the patient is normally awake. Such a requirement is in conflict with the body's normal circadian rhythm for the synthesis of cortisol which suggests that a pharmaceutical such as Nizoral^{®} would best be administered between midnight and three O'clock in the morning. Thus, while conceptually the use of a cortisol synthesis inhibitor (e.g. ketoconazole) in the treatment of metabolic syndrome and diabetes mellitus type II is a significant advance, the therapeutic methods described, inter alia, in US 5,849,740 could be improved both in terms of therapeutic efficacy and convenience of administration.

WO 00/00179 discloses a formulation of oleic acid, hydroxypropylmethylcellulose and PEG 6000; the release profile of the formulation is not disclosed.

In the pharmaceutical industry it is generally recognised that formulations for medical treatment should ideally have a reasonable shelf-life; in other words be stable under normal conditions of storage, i.e. room temperature or 'controlled room temperature' a US standard which means 20-25°C with temporary 'excursions' within the range of 15-30°C, to simulate fluctuating temperatures which may be experienced, e.g. during transportation. Mechanisms exist for storing medicaments at much lower temperatures than room temperature but this will increase the effective cost of the drug.

One approach which might be considered in an attempt to improve storage stability would be to vary the size of the crystalline particles of the active agent, here ketoconazole. Ketoconazole from different manufacturers was tested with particle sizes ranging from 16 µm to 65 µm (these values are for the diameter and are the values at which 90% of the particles in the sample have a smaller diameter). When the dissolution profiles of these formulations were tested it was seen that while a delayed release profile could be obtained, the formulations were unstable after storage for 1 week at 30°C both with the small and the large particle size.

A novel temporal and spatial release profile has now been developed for formulations containing ketoconazole. More specifically, it is now proposed that is desirable to achieve delayed release of ketoconazole and in some cases generation of a reservoir of ketoconazole in the stomach. In further embodiments of the present invention are described formulations comprising ketoconazole which exhibit good stability during storage.

Thus, in one aspect, the present invention provides a pharmaceutical composition comprising ketoconazole or a compound of formula I 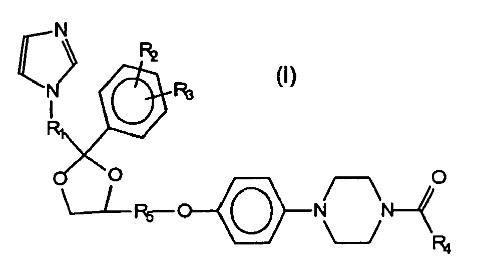 wherein R₁ and R₅ which may be the same or different represent a C₁₋₃ alkyl group, R₂ and R₃ which may be the same or different represent hydrogen, a halogen atom or a methyl group substituted by one or more halogen atoms and R₄ represents the group (CH₂)ₙCH₃ wherein n may be 0, 1 or 2 and a lipid excipient, at least 85% by weight of said lipid excipient consisting of one or more low melting point lipids wherein said low melting point lipid has a melting point less than 30°C, and 1-15% by weight of said lipid excipient consisting of one or more high melting point lipids, wherein said high melting point lipid has a melting point greater than 30°C. Said composition is for controlled release of ketoconazole or the compound of formula I.

Ketoconazole is a particularly preferred cortisol synthesis inhibitor for use in the treatment of diseases characterised by or associated with hypercortisolemia such as metabolic syndrome and diabetes mellitus type II. However this compound is difficult to formulate and administer, in particular due to its low solubility. It is therefore surprising that effective controlled release compositions including ketoconazole as active ingredient can be prepared.

Reference to "ketoconazole" herein includes all four stereoisomers of the racemic cis/cis mixture known in the art as ketoconazole (Rotstein et al., J. Med. Chem. (1992) 35, 2818-2825) and racemic and other mixtures of said stereoisomers. The Cis-2S,4R and Cis-2R,4S isomers are particularly preferred for use in accordance with the present invention. Rotstein teaches a method for preparation of the individual isomers which may be more conveniently prepared by resolution of the available racemic mixture by crystallisation.

The group of compounds represented by formula I above can be considered as derivatives of ketoconazole. Such derivatives having at least 30%, preferably at least 50% or at least 75 or 85% or more of the anti-cortisol activity of ketoconazole itself.

Ketoconazole itself is a compound of formula (I) wherein R₁ and R₅ are CH₂, R₂ and R₃ are Cl and R₄ is CH₃

Further derivatives include salts of ketoconazole itself and of the derivatives discussed above. The formulations preferably contain one or other of the cis isomers of ketoconazole or a racemic or other mixture thereof.

The ketoconazole used in the preparation of the formulations of the invention is preferably in the form of particles of crystalline ketoconazole. The particles may be of different sizes, with diameters typically ranging from 1 to 500 µm more preferably from 5 to 200 µm. These values are determined as the diameter at which 90% of the particles in the sample are no greater than. The particles will preferably be able to form a smooth paste with the release rate controlling substrate(s) and have suitable rheological behaviour for filling gelatine capsules.

By 'controlled release' is meant that the active ingredient is released from the formulation and thus made available for uptake by the body in a regulated manner, this being determined by the release rate controlling substance and interactions between the active ingredient, the release rate controlling substance and the media surrounding the formulation (e.g gastric juice). The compositions of the invention will conveniently be time-specific controlled-release systems, i.e. formulations that are designed to release the active agents in a specific temporal pattern. Release of the majority of active ingredient is not immediate and may, for example, be delayed, sustained or extended. Thus 'controlled release' includes sustained, delayed, extended or modified release, as understood in the art and as may be contrasted with immediate release dosage forms.

The term 'controlled release' is well known in the art and typically relies on a change of state within the formulation after administration which results in release of the active agent from the formulation. The change of state may be caused for example by changes in the temperature, pH, ion concentration etc. in the environment surrounding the composition, e.g. through exposure to an aqueous environment. The change of state may involve a phase transition and/or gelling.

The compositions of the present invention provide a very effective and flexible way of controlling the release of ketoconazole. In a preferred embodiment of the present invention, the in vivo uptake of ketoconazole is sustained for at least 1 hour, preferably at least 2 hours, more preferably at least 3 or 4 hours, particularly at least 6 or 8 hours. In the context of the treatment of Metabolic Syndrome, release will preferably be sustained for 1 to 12 hours, preferably 2 to 12 hours, more preferably 3 to 8 hours e.g. between 4 and 6 hours.

Sustained release will result in a pharmaceutically effective amount of ketoconazole being available for absorption and preferably in the plasma of the patient for the time periods discussed above. In the context of the present invention, a pharmaceutically effective amount (level) will be one which is able to cause a physiologically significant reduction in cortisol activity, typically a physiologically significant reduction in circulating levels of cortisol. Levels of ketoconazole which are pharmaceutically, i.e. physiologically effective and reductions in circulating levels of cortisol which are physiologically significant will vary from patient to patient but can be readily determined, particularly with reference to the parameters of interest in the conditions being treated. Sustained release will preferably result in circulating plasma levels of ketoconazole in excess of 100 ng/ml (typically in excess of 200, 500 or even 1000 ng/ml) for at least 2 hours, preferably 3 or more, more preferably 4 or more, e.g. 4-8 or 4-6 hours.

If the levels of ketoconazole are not uniform in the plasma of the body, it is the circulating plasma level in the relevant organ, e.g. in the adrenal gland which is of most importance.

The *in vivo* release rates of active ingredient from the formulations of the invention can be correlated with the *in vitro* dissolution rates of the compositions when placed in environments which simulate body conditions. Thus, in vitro dissolution of the ketoconazole may be sustained for at least 0.75 hours, preferably at least 1 or 2 hours, more preferably at least 3 or 4 hours, particularly at least 6 or 8 hours as contrasted with about 0.5 hours for the marketed immediate release product of ketoconazole. The Examples include suitable assays for measurement of *in vitro* dissolution.

As discussed above, cortisol levels in the body tend to rise and peak during the night. Preferred formulations of the present invention provide a delayed onset to the release of the ketoconazole. This has the advantage that the composition can be administered while the patient is still awake e.g. before bed-time, between 9p.m. and 11p.m. and yet the release of ketoconazole is delayed and so plasma levels of ketoconazole more closely follow the normal circadian pattern of circulating cortisol levels. In this way, ketoconazole is made available at the most suitable time for counteracting the nightly increase in cortisol synthesis.

Thus, in a preferred embodiment of the present invention, circulating plasma levels of ketoconazole reach pharmaceutically effective levels more than % hour, preferably more than 1 hour, more preferably more than 2 hours after administration of the composition. A practical definition of 'pharmaceutically effective' is provided above in the context of sustained released.

The formulations of the invention will therefore preferably exhibit a 'lag time' between administration and the presence in the plasma of pharmaceutically effective levels. Another way to define and investigate this delayed onset to the release of ketoconazole is to calculate t_{first}, the arithmetic mean between the time of the last sample point before appearance in plasma and the time of the first sample point at which the active agent is detected in the plasma. Thus, for example, if samples are analysed every 20 mins from administration and at 300 mins there is no detectable ketoconazole in the sample but at 320 mins ketoconazole is detected, t_{first} will be 310 mins. t_{first} may vary slightly with the detection method used, and the values discussed herein are derived from the method described by Kay Hay Yuen and Kok Khiang Peh in the Journal of Chromatography B, 715 (1998) 436-440 for determining levels of ketoconazole in plasma. The compositions of the invention will preferably have a t_{first} in excess of ½ hour, preferably in excess of 1 hour, more preferably in excess of 1½ or even 2 hours. The values for t_{first} are preferably mean values, there being a potential for considerable variation from patient to patient.

The delay may conveniently be achieved due to the time taken by the composition to react to its surroundings, e.g. by taking on water or reacting to a change in pH, and undergo a change of state in order to release the active ingredient(s).

It is easily realised by those skilled in the art that the pharmacokinetic parameters such as Cₘₐₓ (peak plasma concentration of ketoconazole), tₘₐₓ (time of peak plasma concentration of ketoconazole) t_{first} (defined above) and bioavailability of the formulations according to the invention depend on e.g. (1) the choice of release rate controlling substance in the formulation, (2) the route of administration and (3) the state of the patient.

Advantageously, the time taken from administration for the plasma ketoconazole concentration to first exceed 100ng/ml will be in excess of 0.5 hour, preferably in excess of 1 hour and more preferably in excess of 2 or 3 hours. As will be discussed in more detail below, this is conveniently achieved following oral administration of the compositions of the invention. Inter-patient variation can be considerable and the above values represent typical values or mean values for a sample of patients.

As well as the time taken for ketoconazole to reach a threshold circulating concentration, the delayed onset and sustained release of the formulations of the invention can be contrasted with standard immediate release dosage forms such as Nizoral^{®} by reference to the time taken for the circulating concentration of ketoconazole to peak. This will typically be more than 2, preferably more than 3, e.g. between 4 and 8 hours after administration.

A typical profile for plasma concentration of ketoconazole administered in a composition according to the present invention has 4 distinct phases. During the first phase, there is minimal release of ketoconazole from the formulation and circulating levels of ketoconazole rise negligibly if at all. In the second phase, there is a fairly steep increase in concentration in the plasma. The third phase is a "plateau" phase where circulating levels remain roughly constant. In the final phase, the circulating level drops to the starting concentration. Phases 3 and 4 may not be distinct, instead there may be a gradual decline in plasma concentration after the peak value has been reached. The presence in the plasma profile of a delayed but then rather rapid increase in ketoconazole concentration represents a preferred feature of the present invention. An assessment of how rapid is the increase in ketoconazole can be made by measuring the time taken from t_{first} to Cₘₐₓ, this will preferably be less than 7 hours, more preferably less than 6 hours, possibly less than 4 hours but a large variation between patients and different formulations may be observed.

In a preferred embodiment, as well as providing a reservoir of ketoconazole in the stomach, the formulations allow for most of the ketoconazole to be absorbed in the upper part of the small intestine, i.e. in the duodenum. Thus, more than 50%, preferably more than 60%, more preferably more than 70% of ketoconazole is taken up in the duodenum.

The "reservoir" of ketoconazole refers to the accumulation of ketoconazole in the stomach, either following release from the formulations of the invention or still present in the retained controlled release formulation. Preferably, the controlled release formulation is retained in the stomach while the ketoconazole, or at least the majority of the ketoconazole, is released into the aqueous phase. The formulation is retained in the stomach due to its physiochemical properties (e.g. size, density, mucoadhesive properties).

Without wishing to be bound by theory, it is believed that the desired release profile is achieved because the ketoconazole is slowly released from the lipid phase into the aqueous phase in the stomach. The aqueous environment in the stomach is at a low pH and part of this will enter the formulation (any gelatine or similar capsule will soon be lost) and the ketoconazole will start to dissolve into this aqueous phase and diffuse out of the formulation through channels formed within the lipid. There is no barrier to the further movement of the ketoconazole dissolved in the aqueous phase and this passes into the small intestine where absorption primarily takes place. The majority of the ketoconazole is released from the formulation in this way and results in a steady flow of the ketoconazole into the upper parts of the small intestine.

After sometime (several hours, preferably 3-8 hours) the formulation comprising the lyotropic lipid phase and residual ketoconazole passes through the gastro-duodenal junction. Preferably no more than 30%, more preferably no more than 20% of the ketoconazole remains within the formulation at this point. The final release of ketoconazole from the controlled release formulation is triggered by the environment of the small intestine. In the small intestine the lipid-based structure is broken down, inter alia by bile salts and lipases, and the residual ketoconazole is then released for uptake.

Two parameters are particularly important to give the desired pharmacokinetic profile, the time the formulation is retained in the stomach and the rate of release of ketoconazole. As described in the Examples herein, these parameters are assessed in vitro by disintegration testing and dissolution testing (simulated gastric fluid).

According to one embodiment, preferably, 4 hours after administration of a single dose, at least 60%, more preferably at least 80% of the ketoconazole from that dose is present in the stomach; after 6 hours at least 50%, more preferably at least 70% of the ketoconazole is still present in the stomach. Devices are discussed herein which provide gastric retention. In a preferred embodiment, at least 60%, more preferably at least 75% of the ketoconazole is released from the formulation in the stomach. These properties result in the formulation of a ketoconazole reservoir in the stomach.

Overall in the formulations of the invention, it preferably takes 6 hours, more preferably 8 hours for 85% of the ketoconazole to be released.

Suitable release rate controlling substances are pharmaceutically acceptable excipients which are capable of modifying the rate at which the ketoconazole becomes available for uptake by the body. The release rate controlling substance is central to modulating the pattern of active agent-release in a predictable fashion. The ability of this substance and/or the formulation as a whole which incorporates the substance to undergo a change in state following administration, typically provides a mechanism which allows gradual and sustained release from the composition of the active agent.

The release rate controlling substance will preferably be in the form of a solid or semi-solid matrix, in which, conveniently, the cortisol antagonist may be dispersed. This can be contrasted with simple capsules, films, coatings and the like which may provide, for a time, a barrier between the active ingredient and the body but are not release rate controlling substances as understood by the skilled man working in this area.

It has been established that the desirable profiles and properties described above can be achieved in a number of different ways. In particular, in order to achieve the desired properties, the formulations contain a lipid excipient, at least 85% by weight of said lipid excipient consisting of one or more low melting point lipids wherein said low melting point lipid has a melting point less than 30°C, and 1-15% by weight of said lipid excipient consisting of one or more high melting point lipids, wherein said high melting point lipid has a melting point greater than 30°C

These formulations release all or most of the ketoconazole in the stomach and advantageously also show mechanical stability in the stomach as monitored using a disintegration bath which can apply mechanical stress to mimic peristaltic movements. In these formulations the largest amount by weight of release rate controlling substance is one or more monoglyceride with long chained fatty acid residues, i.e. having between 6 and 22 carbon atoms, more preferably between 16 and 22 carbon atoms; these monoglycerides are preferably rich in unsaturated fatty acid residues and most preferably comprise all unsaturated fatty acid residues. Other formulations of the invention, such as those with good storage stability, will also comprise such monoglycerides as the largest amount by weight of a release rate controlling substance.

Monoglycerides such as monoolein and monoolein rich monoglyceride blends are especially preferred for use in these compositions of the invention. By monoolein is meant a product which consists predominantly of glyceryl monooleate (also known as glycerol monooleate), although various other glycerides of oleic acid and other fatty acids could be present. Hereinafter references to glyceryl monooleate should be construed also as a reference to monoolein and visa versa. Thus preferably the compositions of the invention will include glyceryl monooleate or glyceryl monolinoleate, glyceryl monooleate being preferred.

However in order to demonstrate the particularly desirable properties discussed above, these formulations also contain 1-15% e.g. 2-10% of a high melting point lipid particularly preferably the formulations contain 2-5% of one or more high melting point lipids. A high melting point lipid is described as one with a melting point greater than (or equal to) 30°C in the final formulation, as for instance determined by DSC. These lipids will preferably be freely soluble (in their liquid state) in melted monoglycerides such as GMO. Suitable lipids are esters of long chained fatty acids, particularly glycerides (mono-, di- or triglycerides) of saturated or predominantly saturated (i.e. no more than one carbon:carbon double bond) long-chained fatty acids. Long chain fatty acids have at least 6 carbon atoms, typically at least 12 carbon atoms and preferably at least 14 carbon atoms. As well as glycerol other compounds containing OH groups may be used to generate phospholipids or waxes which also incorporate long-chain fatty acids as described above.

A preferred group of fatty acids are lauric acid (a triglyceride but not mono- or di-glyceride thereof is suitable) myristic acid, palmitic acid, stearic acid, arachidic acid, behenic acid, lignoceric acid and nervonic acid. The melting point is key to the suitability of a given lipid and this is readily determined experimentally (e.g. by DSC), this may be appropriate, for example when assessing the suitability of mixed di- or triglycerides where one fatty acid chain is saturated and another is unsaturated. Fatty acid chain length impacts on melting point as well as the presence or absence of carbon:carbon double bonds and so this will also be taken into account when selecting suitable lipids.

More generally, lipids (particularly esters, e.g. citric acid esters of the mono and di-glycerides discussed above) with a high melting point, i.e. greater than 30°C (for example 35-45°C) in the final formulation as for instance determined by DSC (differential scanning calorimetry), between 35-45°C give enhanced mechanical stability.

Example 16 herein provides a suitable method for confirming how much of a given high melting point lipid is appropriate and indeed confirming the suitability of a candidate lipid.

The % values given above refer to the amount by weight of a high melting point lipid component which is added to the other excipients in the formulation. The percentages exclude the ketoconazole itself. This is important as commercially available lipid excipients do not currently comprise 100% of a single chemical species and thus the low melting point lipids such as GMO which constitute the primary excipient in many of the formulations of the present invention will typically comprise small amounts of diglycerides and saturated fatty acids as well as glycerol monooleate (see Table 2). Some of these additional components will be high melting point lipids and the above percentages are based on RyloMG 19 Pharma produced by Danisco as the primary low melting point excipient. RyloMG 19 Pharma itself comprises about 5% of high melting point lipids.

It is recognised that alternative primary excipients may be chosen and that in the future the purity of such excipients may be improved. In which case, the protocol of Example 16 may be used to select a suitable amount of high melting point lipid which should be added to the primary excipient to give the desired two phases after heating to 35°C for 18 hours. This ability not to melt to a liquid under these conditions is key to the advantageous stable properties of the formulations of the invention. The most appropriate amounts should still fall within the broadest range discussed above and typical total high melting point lipid components in the formulation will be 1 to 15%, more preferably 6 to 15%, e.g. 7-11%.

Therefore in an alternative embodiment, the present invention provides a pharmaceutical composition as defined above with a sufficent quantity of one or more high melting point lipid excipients to provide a liquid and a solid phase within said formulation after heating to 35°C for 18 hours. Details of this melting test are found in Example 16.

The addition of another solid which acts in the same way to enhance storage stability by hindering the settling of ketoconazole crystals at the bottom of the formulation (e.g. silicon oxide) may be useful in improving the storage stability of the other formulations described herein.

In a particularly preferred embodiment of the present invention, the formulations form a non-lamellar lyotropic liquid crystalline phase, preferably cubic or reversed hexagonal, when in contact with an aqueous media, e.g. as results from oral administration of the dosage form. Such phases are well studied in the art and a useful review is found in EP 0126751. The matrix is able to provide a controlled-release of the ketoconazole.

The primary release rate controlling substance in these formulations are low melting point lipids, such as the low melting point monoglycerides which are discussed in some detail above. In this context, a low melting point is defined as being less than 30°C in the final formulation, as determined e.g. by DSC. These form non-lamellar liquid crystalline phases with water at body temperature. They are typically 'purified' monoglycerides which have been obtained through molecular distillation (e.g. through distillation of tall oil fatty acids).

The addition of a high melting point lipid, has surprisingly been found to improve the storage stability of the formulations without adversely affecting the desired release profile. In other words a more robust formulation is generated in which the release profile of the freshly prepared formulation is maintained after periods of storage. The conditions of storage are in accordance with the US standard of controlled room temperature', 20-25°C with temporary 'excursions' within the range of 15-30°C. The formulations of the invention are preferably stable during storage under these conditions for at least 3 months at 25°C with temporary 'excursions' for at least 1 day, preferably at least a week or even 1 month or more.

The formulations of the invention can be considered stable if their release profile, as measured by the *in-vitro* dissolution tests described in the Examples hereto, is substantially the same after one week, preferably one month, at 'controlled room temperature' as it is when it has been freshly prepared. The profiles can be considered 'substantially' the same when the average of the differences in % of ketoconazole dissolution for each time point is 10% or less as between freshly prepared and stored, or as shown by other standard techniques in this field. These stable formulations comprise a preferred aspect of the present invention and preferably also provide a reservoir of ketoconazole in the stomach as described herein. The formulations are able to give controlled release of the ketoconazole.

The two lipid components of the formulation of the invention are considered release rate controlling substances and the formulation as a whole provides for the controlled release of ketoconazole. These terms are discussed above. The formulations typically comprise from 5-30 % by weight of ketoconazole, preferably 15-25% by weight.

High and low melting point lipids are defined above and suitable examples give. Also, as discussed above the percentages referred to are in terms of those components added to the excipient mix, it being understood that GMO (commercially available as Rylo MG 19 Pharma) - a low melting point lipid - will also contain small amounts of other lipids. These lipids may not all be classed as low melting point lipids within the meaning of the present invention. The word "consisting of" should be interpreted with is in mind.

Those working in the fields of pharmaceutical manufacture are well aware of the difficulties of obtaining pure substances, particularly pure lipids of the type used in the formulations of the present invention. Nevertheless a lack of purity in the starting materials is not a hindrance to the working of the invention and the Examples herein provide suitable tests for analysing the suitability of a given excipient or combination of excipient.

In one further preferred embodiment an acid may be included in the formulation which will cause a local decrease in the pH in the duodenum and thereby increase the absorption of the ketoconazole.

In a further alternative, the formulation is prepared such that a significant amount of ketoconazole is unreleased when the formulation passes through the gastroduodenal junction but on exposure to the duodenal environment, e.g. the raised pH, bile or local enzymes, the formulation undergoes accelerated disintegration and so ketoconazole is released for absorption.

The compositions may also comprise further active ingredients as well as ketoconazole. Such additional active ingredients will be selected dependent upon the disease/condition being treated and the patient themselves. Growth hormone, testosterone and oestrogen (or analogous thereof or agents which mimic their activity) may conveniently be co-administered with a ketoconazole in the treatment of metabolic syndrome. Co-administration does not require all components to be present in a single formulation as the same route of administration may not be appropriate in all cases.

Further ingredients acting as release rate controlling substances may be added as well as other pharmaceutically acceptable carriers, stabilisers and formulation modifiers. Such are well known to the man skilled in the art and include pH modifiers, antioxidants and completing agents and are exemplified by calcium chloride dihydrate, polyvinyl alcohol, titanium dioxide, tricalcium phosphate, sodium chloride, hydrochloric acid, galactomannan, scleroglucan, citric acid, NaHPO₄ and trisodium citrate dihydrate.

Cortisol overactivity has been implicated in a large number of medical conditions and the compositions of the invention may be used to treat any of these. Therapeutic use of cortisol lowering pharmaceuticals has been considered for the treatment of stress (Med. Hypothesis 13, 31-44, 1984) and anti-cortisol therapy has been suggested for the treatment of an extensive list of diseases such as Cushing's disease/syndrome and depressive diseases, (Psychoneuroendocrinology 22 (suppl. 1) S3-10, 1997). Further diseases which may be treated with the compositions of the invention include androgen related diseases such as prostate cancer, hirsutism and polycystic ovary syndrome. Thus in a further aspect of the present invention is provided a composition according to the invention as defined herein for use in the treatment of any condition related to elevated cortisol activity and/or androgen activity. Suitable conditions will generally be those where elevated circulating levels of cortisol and/or androgens are at least partly responsible for observed symptoms or constitute a risk factor for other conditions.

However, the compositions of the invention are particularly suitable for the prevention or treatment of metabolic syndrome or diabetes mellitis type II or symptoms and complications thereof.

"Treatment" refers to total or partial alleviation of at least one of the symptoms associated with the disease or complications thereof and reference to metabolic syndrome and diabetes mellitus type II may be construed as a reference to the symptoms and complications associated therewith. In a further aspect, the invention provides a method of treating metabolic syndrome or diabetes mellitis type II and symptoms and complications associated therewith which method comprises administering a composition according to the present invention as defined herein. The compositions of the invention are of use in the treatment of mammals, particularly humans.

While the formulations of the invention may be administered by any route known to the skilled man, e.g. oral including buccal and sublingual, transdermal, transmucosal including nasal, pulmonary, rectal, vaginal or parenteral (including intravenous, subcutaneous or intramuscular), oral routes are preferred.

Suitable methods for preparing compositions for oral administration are known to the skilled man. Formulations may be presented in tablet, capsule, powder, syrup, solution or suspension form, tablets and capsules being preferred. The compositions of the invention, which are preferably in solid or semi-solid form, may conveniently be covered in a gelatin or similar capsule for oral administration. Capsules may be hard such as the gelatin capsule of size 000, 43.000 Capsugel (Bornem, Belgium) or soft, hard gelatin capsules being preferred. Suitable encapsulation and tableting techniques are described by Takada K. et al in Encycl. Controlled Drug Deliv. (1999) 2 698-728. Generally, the capsules function merely as a container for the composition of active ingredient and release rate controlling substance. The capsule will become permeable/disintegrate or detach soon after administration of the dosage form, typically in 5-15 mins and is therefore not acting as a release rate controlling substance as defined herein.

The compositions may be formulated by any of the methods known in the art and suitable methods are described in the Examples. Conveniently, a homogenous dispersion of the ketoconazole and release rate controlling substance is prepared above the melting point of the composition which may then subsequently be solidified. Alternatively, the release rate controlling substance may be frozen and then crushed to a fine powder before being mixed with the ketoconazole and any other excipients in dry form to make a homogenous formulation.

Thus, in a further aspect, the present invention provides a method of making a composition for controlled release of a ketoconazole, which method comprises mixing said ketoconazole with a release rate controlling substance, preferably to provide a homogeneous formulation.

The invention will be further described with reference to the following Examples in which:
Figure 1 shows the dissolution profile for ketoconazole in HPMC;
Figure 2 shows the dissolution profile for ketoconazole in sodium alginate (±NaHCO₃);
Figure 3 shows the dissolution profile for ketoconazole in various alginic acid based formulations;
Figure 4 shows the dissolution profile for ketoconazole in GMO and or Carbopol; and
Figure 5 shows the dissolution profile for ketoconazole in GMO/citric acid/diglyceride.
Figure 6 shows the effect on the dissolution of ketoconazole of a lipid with a high melting point.
Figure 7 shows the dissolution profiles for formulations 5-8 of Table 3, which contain 100% GMO or 98% GMO and 2% of a high melting component with one or other of the cis isomers of ketoconazole (KC) or a racemic mixture thereof. 'Sieved' means that the KC contains crystals with a size less than 100 µm.
Figure 8 shows the effect on the dissolution of ketoconazole of varying amounts of GMS, as shown in Table 4.
Figure 9 shows the dissolution profile of the polymer containing formulations of Table 5.

Throughout the following examples, unless otherwise indicated, "KC" refers to a racemic mixture of the two cis isomers of ketoconazole and cis-1 is the 2R, 4S isomer.

### Example 1 (Reference Example)

### Cis-1/ Hydroxypropyl-methylcellulose (HPMC)

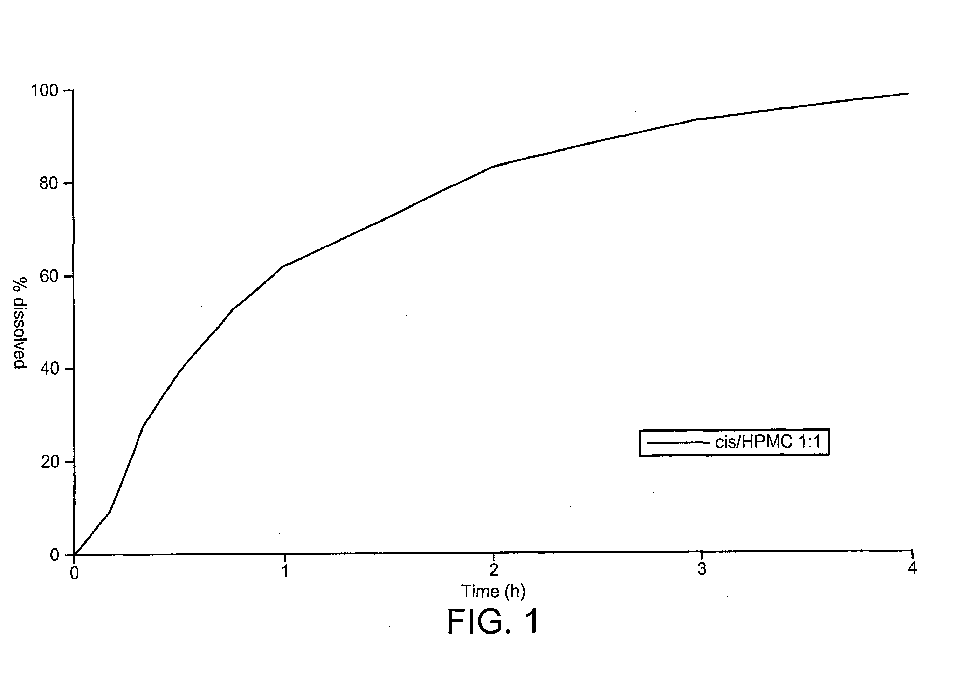

Enough water was added to a dry mixture of cis-1 (50mg) and neutral polymer HPMC, (USP qual. Sigma) (50mg) to swell to a highly viscous gel where the drug powder was suspended. The polymer/ drug was thereafter put in a freeze dryer and the water was removed. The resulting polymer: cis mixture (1:1) was put in a hard gelatine capsule (size #0). Dissolution test in simulated gastric fluid (SGF) without pepsin (see USP 23) was performed and the results are shown in Figure 1. The dissolution profile was obtained with a USP Apparatus I at 100rpm and 37°C by monitoring the absorbance at 254 nm in 500 ml SGF as a function of time.

### Example 2

KC (200 mg/g) was dispersed in one quality of glyceryl monooleate (GMO) with low diglyceride (GDO) content (3.2%) and one with a higher diglyceride content (9.0%). Both formulations (0.5 g) were put into capsules which were tested in an apparatus for investigation of disintegration properties (to mimic peristaltic movement) in aqueous media of tablets and capsules as described in Eu Ph section 2.9.1. The sample was filtrated (0,2 µm) before the amount of KC released was monitored by measuring the absorbance at 254 nm in 700 ml simulated gastric fluid, without pepsin (Gastric fluid, simulated TS, see USP 23) after 0.5 h, see table 1.

### Example 3 (Reference Example)

### Cis-1/GMO/Sesame oil

Cis-1 (200mg) was dispersed in melted GMO (700mg) with low diglyceride content. 100mg of a triglyceride (sesame oil), liquid at room temperature, was added and the formulation was stirred together until a smooth consistency was obtained. The formulation (1g) was filled in hard gelatine capsule (size #000) and stored in refrigerator.

### Example 4

### Cis-1/ GMO/ Akocote

Cis-1 (200mg) was dispersed in melted GMO (700mg) with low diglyceride content. 100mg of a melted triglyceride (Akocote RT, Karlshamns), solid at room temperature, was added. Prepared as in Example 3.

### Example 5

### Cis-1/ GMO/ Glycerol stearyl citrate

Cis-1 (200mg) was dispersed in a melted GMO (700mg) with low diglyceride content. 100mg of the melted glycerol stearyl citrate (Imwitor 370, Hüls AG) was added. Prepared as in Example 3.

Examples 3-5 were subjected to the same disintegration test as the products of Example 2 and the results are shown in the table below.

**Table 1. Results from the disintegration tests**

| **Lipid in KC or cis formulation** | **Amount KC or Cis released, 0.5 h** |
|---|---|
| GMO with 3.2 % GDO | 93 % w/w |
| GMO with 9.0 % GDO | 53 % w/w |
| GMO/ Sesame oil | 89 % w/w |
| GMO/ Akocote RT | 59 % w/w |
| GMO/ Glycerol stearyl citrate | 60 % w/w |

### Example 6 (Reference Example)

### Cis-1/ GMO/ Alginic acid/ NaHCO3

460 mg of melted GMO (with low diglyceride content) was added to a dry powder mixture of cis-1 (200mg), alginic acid (Kelacid, Kelco)(250mg) and NaHCO3 (90mg). The formulation was stirred until a smooth consistency was obtained. The formulation was filled in hard gelatine capsule (size #000) and a dissolution test in SGF was made as in Example 1. By using alginic acid together with sodium bicarbonate in a formulation a very viscous gel is formed in an acidic environment. In the stomach carbon dioxide is released from the formulation making a low density formulation. This formulation and KC:GMO (20:80) were allowed to swell in SGF first for 1 hour before being transferred into a beaker with a media (acetonitril) with lower density (0.786 g/cm3) than SGF. In this buoyancy test the formulation with alginic acid floated whereas the original formulation of KC/GMO sank.

### Example 7(Reference Example)

### Cis-1/ Alginic acid/ NaHCO3

Cis-1, (120mg), alginic acid, (354mg) and NaHCO3 (126mg) were mixed together. The formulation was filled in hard gelatine capsule (size #000) and a dissolution test in SGF was made.

### Example 8 (Reference Example)

### Cis-1/ Alginic acid

Adequate amount of water was added to a dry mixture of cis-1 (50mg) and the polymer alginic acid (50mg). The polymer was allowed to swell to viscous consistency and the water was thereafter removed in a freeze dryer. The resulting polymer: cis mixture (1:1) was put in a hard gelatine capsule (size #0) and a dissolution test in SGF was made.

### Example 9 (Reference Example)

### Cis-1/ Sodium alginate/ NaHCO₃

Cis-1, (120mg), sodium alginate (Keltone LVCR, Kelco) (354mg) and NaHCO₃ (126mg) was mixed together. The formulation was filled in hard gelatine capsule (size #000) and a dissolution test in SGF was made.

### Example 10 (Reference Example)

### Cis-1/ Sodium alginate

Adequate amount of water was added to a dry mixture of cis-1 (50mg) and the polymer sodium alginate (50mg). The polymer was allowed to swell to viscous consistency and the water was thereafter removed in a freeze dryer. The resulting polymer: cis mixture (1:1) was put in a hard gelatine capsule (size #0) and a dissolution test in SGF was made.

### Dissolution profiles from Examples 6-10:

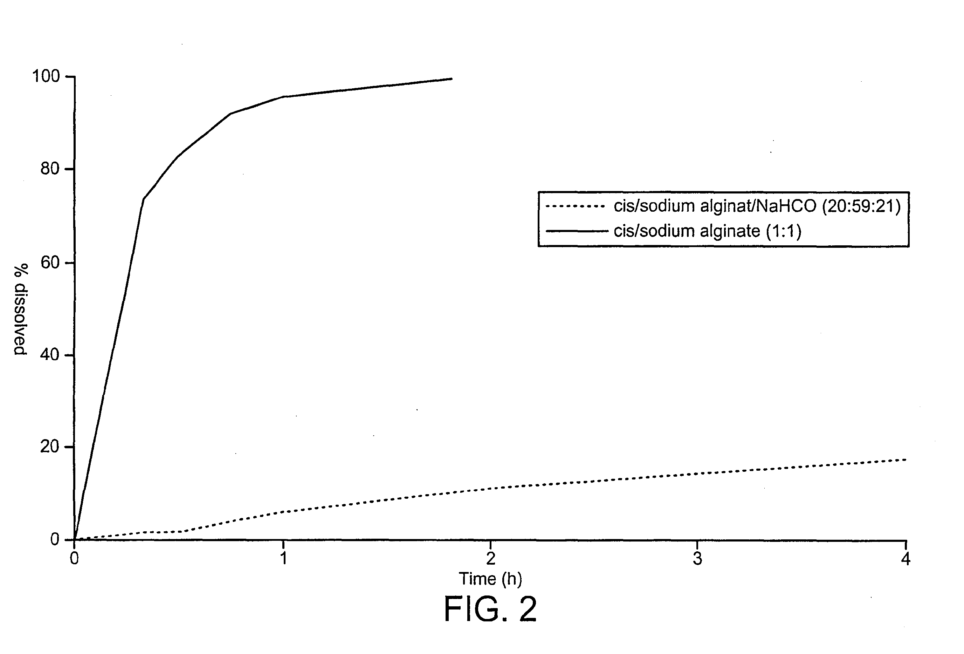

### Formulations with sodium alginate, Figure 2

The mixture cis/ sodium alginate gives a relatively fast dissolution profile, nearly as fast as a immediate release product since 83% was released after 0.5 hour. (An accepted definition of immediate release is release 85% of active agent after half an hour). When NaHCO₃, was added a much slower profile was obtained, which was surprisingly. Only 2% was released after 0.5 hour and 18% after 4 hours.

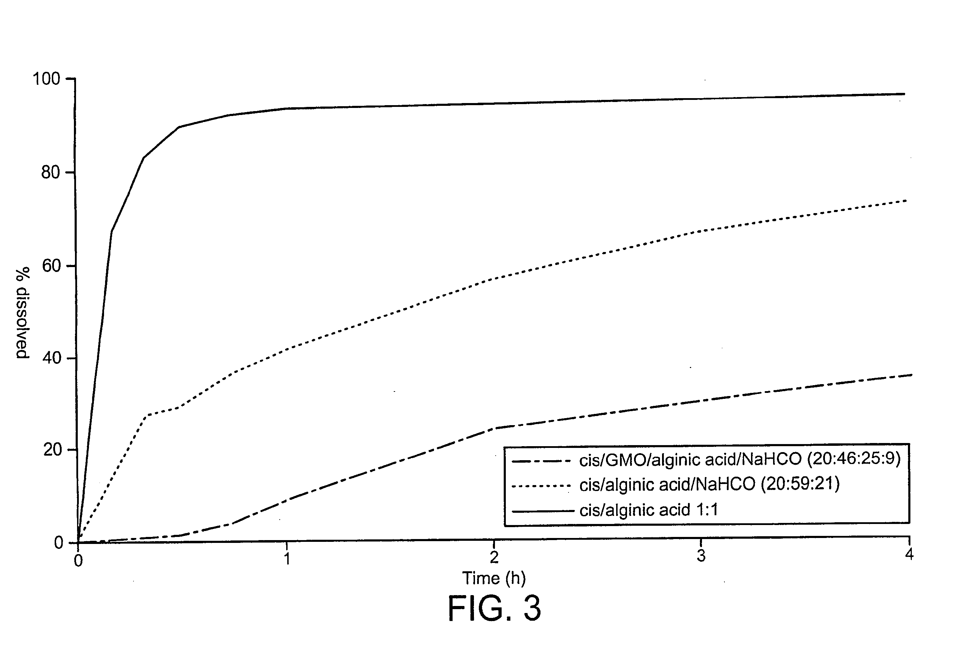

### Formulations with alginic acid, Figure 3

The mixture cis/ alginic acid gives a fast release (IR) but also in this case the release profile was slowed down by adding NaHCO₃, (30% after 0.5 hour) although not as much as in the combination with the sodium alginate. By adding GMO to the mixture cis/ alginic acid/ NaHCO3, the release was slowed down further, releasing only 2% after 0.5 hour and 25% after 4 hours.

### Example 11 (Reference Example)

### Cis-1/ Carbopol 934P

50mg of cis-1 was dissolved in 2.5ml methanol. 50mg of carbopol (BFGoodrich) was added to the mixture whilst stirring. The methanol was removed in a freeze dryer and the resulting polymer: cis mixture (1:1) was grained in a mortar, put in hard gelatine capsule (size #0) and a dissolution in SGF was made.

### Example 12 (Reference Example)

### Cis-1/ GMO/ Carbopol 934P

600mg of melted GMO (with low diglyceride content) was added to 200mg of cis-1 and 200mg of carbopol. The formulation was stirred until a smooth consistency was obtained. The formulation was filled in a hard gelatine capsule (size # 000) and a dissolution test in SGF was made.

### Dissolution profiles from Examples 11 and 12:

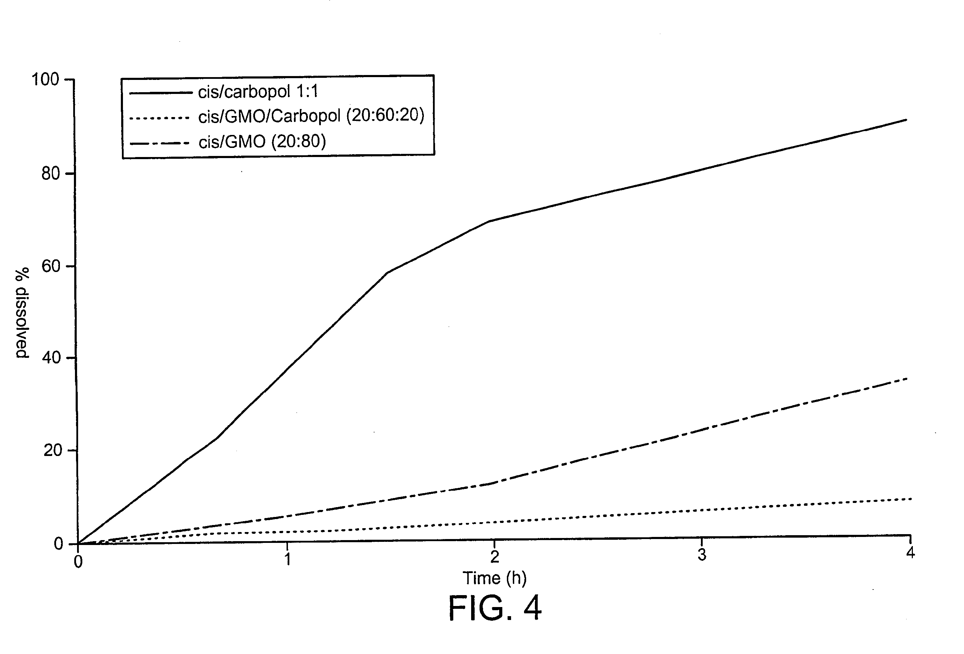

The results of the release profiles are presented in Figure 4 and it is clear that the carbopol effects the release profiles the desired way. The formulation with cis/ carbopol has after 0.5 hour released only 37%. By adding GMO to the cis/ carbopol, the dissolution profile was slowed down drastically (2% after 0.5 hour and 8% after 2 hours). The cis/GMO curve is plotted for comparative purpose and has after 0.5 hour released 6% and 34% after 2 hours.

### Example 13 (Reference Example)

### KC/ GMO/ Citric acid

800mg of melted GMO (with 9% of diglyceride) was added to 100mg of KC and 100mg of citric acid. The formulation was stirred until a smooth consistency was obtained. The formulation was filled in a hard gelatine capsule (size # 000) and a dissolution test in SGF was made.

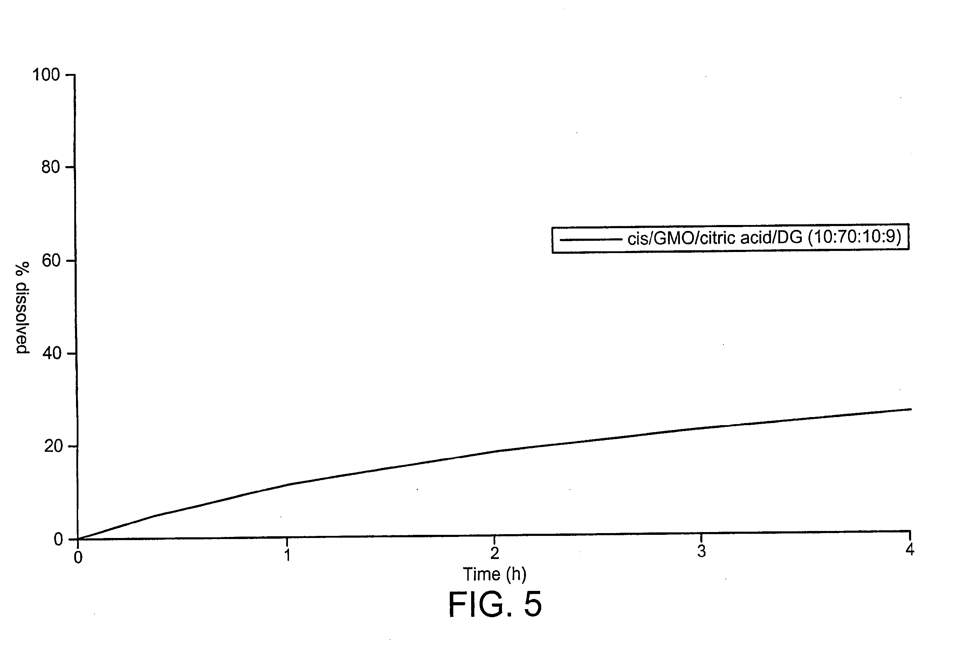

The dissolution profile is presented in Figure 5 and gives the desired delayed profile in the stomach, with 6% released after 0.5 hour and 26% after 4 hours.

In the following examples abbreviations for certain chemical components are used and these are defined in Table 2 below.

**Table 2**

| Raw material | Abbreviation | Supplier | Product information |
|---|---|---|---|
| Ketoconazole | KC_{Jal} | Jal Healthcare | |
| Ketoconazole | KC_{Cl} | Chemo Iberica | D(<0,9) 63 µm |
| Ketoconazole | Rec1 | Recordati | D(<0,9) 16 µm |
| Ketoconazole | Rec2 | Recordati | D(<0,9) 65 µm |
| 2R, 4S-Ketoconazole | KC Cis-1 | Clauson-Kaas | |
| 2S, 4R-Ketoconazole | KC Cis-2 | Clauson-Kaas | |
| Akofine S | | Karslhamns | hydrogenated triglyceride |
| Polyacrylic acid | PAA_{high} | Aldrich | Mw 450 000 |
| Polyacrylic acid | PAA_{low} | Aldrich | Mw 2 000 |
| Polyethylene glycol 4000 | PEG4000 | Merck | Ph.Eur. |
| Poloxamer (Lutrol F127) | | BASF | copolymer of ethylene oxide and |
| | | | propylene oxide, USP/NF |
| Rylo MG 12 | GMLa | Danisco | glycerol monolaurate |
| Rylo MG 18 | GMS | Danisco | glycerol monostearate, monoester >90% |
| Rylo MG 19 Pharma | GMO | Danisco | glycerol monooleate monoglyceride 95.1% |
| | | | diglyceride 4.1% |
| | | | C_{18:1} min. 88% |
| | | | 5% saturated fatty acids |
| | | | 93.9% unsaturated fatty acids |

### Example 14

### Effect of addition of lipids with a higher melting point than glycerol monooleate on release and storage properties of KC - GMO formulations.

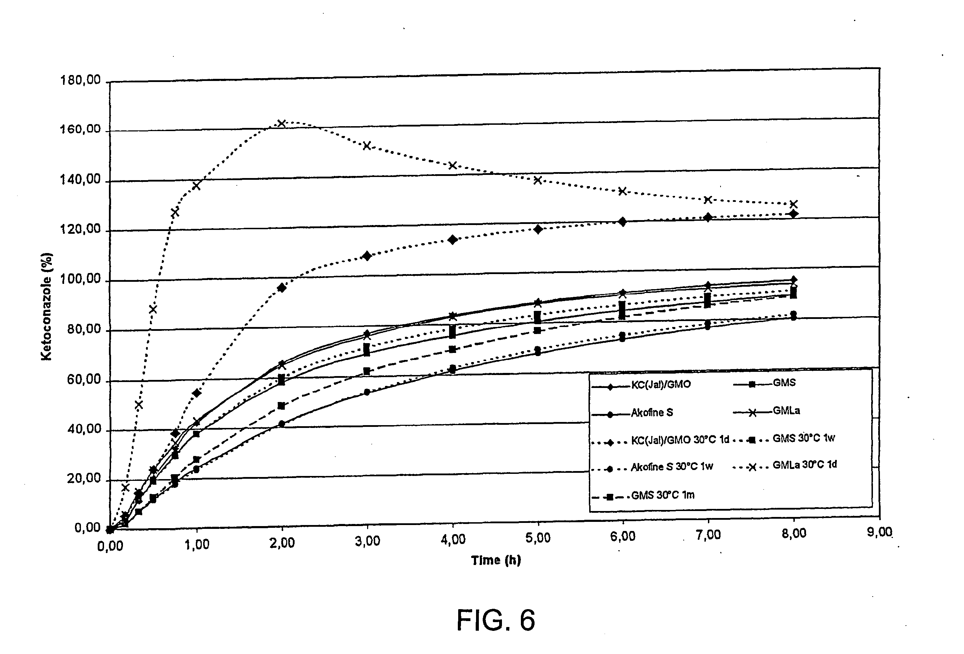

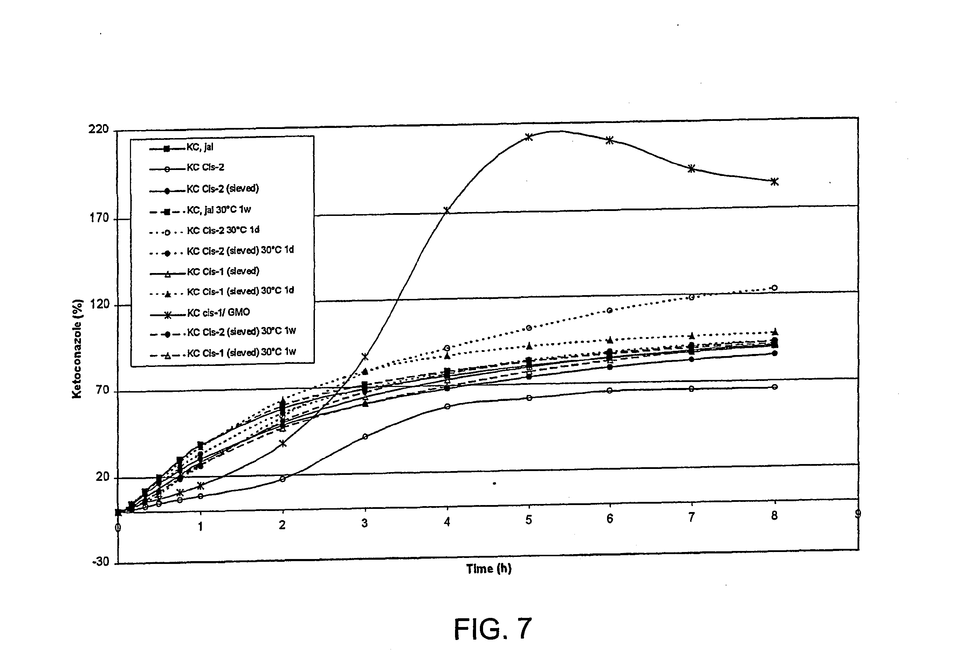

Tables 3 and 4 show the effect of changes in composition of the lipid excipients on release profile and stability (storage at 30°) for KC - lipid formulations. The tables summarise released amount of KC as a function of time for freshly prepared samples and samples stored at 30°C, see also figures 6 and 7.

Batches of 5-25 g were prepared and filled in capsules in the following manner.
- The lipids were melted at a minimum temperature, normally 40°C for GMO. When a high-melting lipid was added, a temperature as high as (70°C) was sometimes necessary in order to get a homogenous liquid oil. The lipids were melted and thoroughly stirred until a homogenous oil was formed.
- The ketoconazole compound was added (20 % w/w) to the mixture of the melted lipids.
- All the ingredients were mixed manually with a spatula until a homogenous paste was obtained.
- Finally, the formulation was put in a syringe and 500 mg of the formulation (representing 100 mg KC per unit) was filled into capsules. Occasionally, the formulation was reheated for a few minutes at 40°C to control the flow properties of the semisolid paste.

The capsules were placed in a refrigerator for at least 12 hours before they were tested. The formulations were intended for gastric release, therefore simulated gastric fluid (SGF, pH 1.2) was used as dissolution media. USP apparatus 1, rotating basket, was used for studying in vitro dissolution of capsules filled with the various formulations (0,5 g). An ERWEKA DT70 apparatus with automatic sampling was used, and the KC release was monitored on-line by UV spectroscopy. It is worth noticing that some values in the tables below report a higher release of KC than 100 %. This is related to disintegration of the formulation which induces formation of lipid particles which obscure the light beam of the UV spectroscopy used to monitor KC release.

The data summarised in table 3 shows that formulations 1-4 give a delayed release of KC. It is also clear from the table that formulations 2 and 3, with an added second high melting lipid component, exhibit increased physical stability over a prolonged time compared to formulation 1 which just contains GMO. This formulation shows phase separation (due to sedimentatiomn of KC particels) after less than one day storage at 30°C. That addition of a low melting point saturated lipid GMLa (#4) to the KC/GMO formulation does not improve the physical stability during storage at 30°C is also apparent from data in table 3.

Table 3 also shows that formulations of both cis isomers of KC can be made with increased thermal (storage) stability. The results also show that the particle size of the active substance affects the release profile ('sieved' KC means KC crystals with a diameter of <100 *µ*m). This may be a useful tool in providing a desired release profile.

**Table 3. Effect of composition of the lipid excipient on release profile and stability (storage at 30°) for KC - lipid and KC (Cis-1, Cis-2) - lipid formulations. KC to lipid ratio of 20 to 80 by weight (see also figures 6 and 7). The lipid excipient contained 98 % w/w GMO and 2% of a high melting component (see, table 2 for abbreviations and product information of the lipids).**

| | #1 | #2 | #3 | #4 | #5 | #6 | #7 | #8 |
|---|---|---|---|---|---|---|---|---|
| Time (h) | KC_{Ja1}/ GMO | KC_{Ja1} /GMO /GMS | KC_{Ja1}/GM O/Akofine S | KC_{CI}/ GMO /GMLa | KC Cis-2/ GMO /GMS | KC Cis-2 (sieved)/ GMO /GMS | KC (Cis-1 (sieved)/ GMO/G MS | KC (Cis-1) /GMO |
| | **freshly prep.** | **freshly prep.** | **freshly prep.** | **freshly prep.** | **freshly prep.** | **freshly prep.** | **freshly prep.** | **freshly prep.** |
| **0.5** | 20,5 | 20,0 | 12,0 | 23,8 | 4,5 | 16,4 | 13,8 | 7,3 |
| **1** | 42,5 | 38,1 | 24,2 | 43,3 | 8,6 | 30,1 | 28,29 | 14,9 |
| **2** | 65,6 | 58,3 | 41,7 | 64,7 | 17,8 | 48,3 | 49,82 | 38,1 |
| **4** | 83,5 | 75,5 | 61,5 | 83,0 | 57,5 | 68,2 | 73,41 | 171,7 |

| | **1d** | **1w** | **1w 30°C** | **1d** | **1d 30°C** | **1d** | **1d** | |
|---|---|---|---|---|---|---|---|---|
| | **30°C** | **30°C** | | **30°C** | | **30°C** | **30°C** | |
| **0.5** | 23,9 | 19,2 | 11,7 | 88,3 | 10,2 | 16,6 | 18,2 | |
| **1** | 54,7 | 38,0 | 23,4 | 137,8 | 26,0 | 32,9 | 36,9 | |
| **2** | 96,1 | 60,0 | 41,3 | 161,9 | 53,6 | 55,7 | 63,3 | |
| **4** | 113,8 | 78,2 | 62,6 | 143,8 | 91,5 | 77,7 | 87,4 | |

| | | **1m** | | | | **1w** | **1w** | |
|---|---|---|---|---|---|---|---|---|
| | | **30°C** | | | | **30°C** | **30°C** | |
| **0.5** | | 12,5 | | | | 11,7 | 13,4 | |
| **1** | | 27,6 | | | | 26,5 | 27,1 | |
| **2** | | 48,5 | | | | 50,8 | 46,9 | |
| **4** | | 69.8 | | | | 76,9 | 69,6 | |

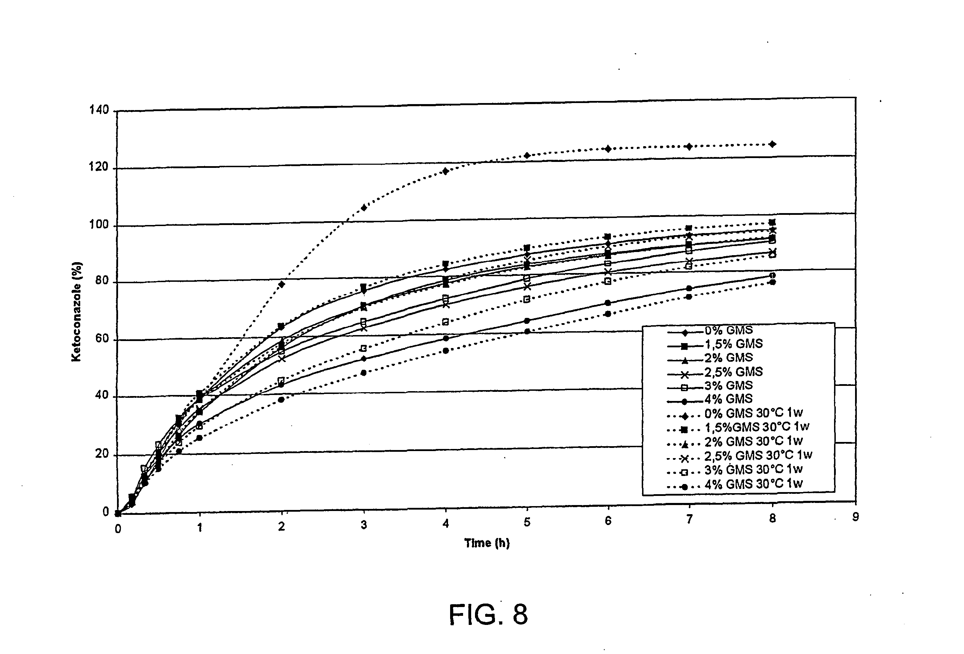

**Table 4. Effect of composition of the lipid excipient on release profile and stability (storage at 30°) for KC - lipid formulations at a ratio of 20 to 80 by weight (see, figure 8). The lipid excipient contained GMO and varying amounts of GMS - a high melting point component (see, table 2 for abbreviations and product information of the lipids).**

| Time (h) | 0% | 1,5% | 2,0% | 2,5% | 3,0% | 4,0% |
|---|---|---|---|---|---|---|
| | freshly prep. | freshly prep. | freshly prep. | freshly prep. | freshly prep. | freshly prep. |
| 0.5 | 18,6 | 17,2 | 21,2 | 20,4 | 23,4 | 18,6 |
| **1** | 39,2 | 34,1 | 39,0 | 35,4 | 38,7 | 30,3 |
| **2** | 63,4 | 56,4 | 58,9 | 52,4 | 55,0 | 43,4 |
| **4** | 82,6 | 78,6 | 77,6 | 70,3 | 72,3 | 58,6 |

| | **1w 30°C** | **1w 30°C** | **1w 30°C** | **1w 30°C** | **1w 30°C** | **1w 30°C** |
|---|---|---|---|---|---|---|
| **0.5** | 20,5 | 20,9 | 21,2 | 17,9 | 16,7 | 15,2 |
| **1** | 38,9 | 40,8 | 38,4 | 34,9 | 29,3 | 25,4 |
| **2** | 78,2 | 63,8 | 57,8 | 56,9 | 44,8 | 38,0 |
| **4** | 117,1 | 84,4 | 77,1 | 78,9 | 64,2 | 54,2 |

Tables 3 and 4 show that by using a purified monoglyceride with added high melting lipids as excipient to formulate KC and its cis isomer, both release rate from the lyotropic non-lamellar liquid crystalline phase and thermal stability of the formulation can be controlled.

### Example 15 (Reference Example)

### Formulations of KC with polyacrylic acid and other polymers

Table 5 shows that the release profile of KC in acid media may be modified by incorporation of polyacrylic acid of different molecular weight PAA_{low} (Mw 2000) and PAA_{high} (Mw 450 000). The formulations were prepared by mixing KC and poyacrylic acid in a ratio of 2:8 in a mortar. The mixture was pressed to a tablet (10mm diameter) and the release profiles for the formulations are summarised in table 5.

Two other polymer formulations are also shown in the table, one incorporating water soluble polyethylene glycol (PEG 4000) with an expected rapid release and one incorporating a surface active polymer, a copolymer of polyethylene glycol and polypropylene glycol exhibiting a delayed release. These formulations are prepared by dispersion of KC in a melted polymer in a ratio of 2:8.

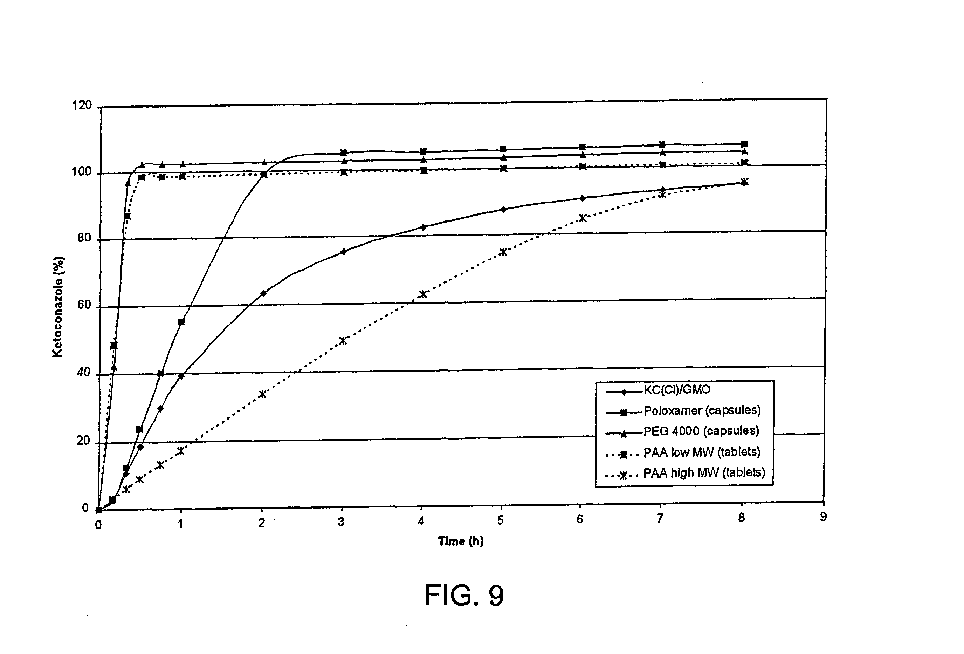

**Table 5. Formulations with polymers (see figure 9)**

| Time (h) | KC_{Jal}/Poloxamer (20/80) | KC_{Jal}/PEG4000 (20/80) | KC_{Jal}/PAA_{high} (20/80) | KCⱼₐₗ/PAA_{low} (20/80) |
|---|---|---|---|---|
| 0.5 | 23,6 | 102,5 | 9,1 | 98,7 |
| 1 | 55,1 | 102,5 | 17,2 | 98,8 |
| 2 | 99,2 | 102,8 | 33,6 | 99,1 |
| 4 | 105,4 | 103,2 | 62,5 | 99,7 |

### Example 16

### Selection of lipid mixtures for KC formulations based on their melting behaviour.

An increased physical stability over a prolonged time at elevated temperatures appears to be related to the existence of a crystalline lipid phase at that temperature. A test can be performed based on visual inspection of the samples that reveals the amount and type of lipid that is suitable as a high-melting component to increase physical stability of lipids forming non-lamellar liquid crystalline phases at body temperature.

The samples were prepared by melting and mixing the liquid lipids and then letting them solidify in a fridge (2h at 4C). Details of a suitable melting and mixing method are described in Example 14.

Table 6 below shows that both GMO and GMO/GMLa melt and form a liquid in less than two hours at 35°C, while the samples containing lipids with longer chain length (with higher melting points) still contain lipid crystals after 18 h at 35°C. As discussed herein, it is understood that during storage samples may be exposed to temperatures as high as 35°C for short periods, e.g. 1 day.

The experiments show that compositions 3,4 and 5 are suitable for formulations of dispersed KC crystals in a semisolid lipid matrix.

**Table 6: Melting behaviour of lipid mixtures, 98 % w/w GMO and 2 % w/w of a second monoglyceride (see table 7 for abbreviations), as monitored by visual inspection of samples. The phase properties as a function of time at 35°C are reported in the table. The expression "two phases" denotes the coexistence of one liquid and one solid phase.**

| **Composition#** | **sample content** | **0min** | **30min** | **70min** | **105min** | **18h** |
|---|---|---|---|---|---|---|
| 1 | GMO | solid | two phases | two phases | liquid | liquid |
| 2 | GMO/GMLa | solid | liquid | liquid | liquid | liquid |
| 3 | GMO/GMP | solid | two phases | two phases | two phases | two phases |
| 4 | GMO/GMS | solid | two phases | two phases | two phases | two phases |
| 5 | GMO/GMB | solid | two phases | two phases | two phases | two phases |

**Table 7**

| Abbreviations used in Example 16: | | | |
|---|---|---|---|
| **Commercial name** | **Abbreviation** | **Supplier** | **Information** |
| Rylo MG 19 | GMO | Danisco | Glycerol monooleate |
| Rylo MG 12 | GMLa | Danisco | Glycerol monolaurate |
| Rylo MG 16 | GMP | Danisco | Glycerol monopalmitate |
| Rylo MG 18 | GMS | Danisco | Glycerol monostearate |
| Dimodan MB90 | GMB | Danisco | Glycerol monobehenate |

## Claims

1. A pharmaceutical composition comprising ketoconazole or a compound of formula I 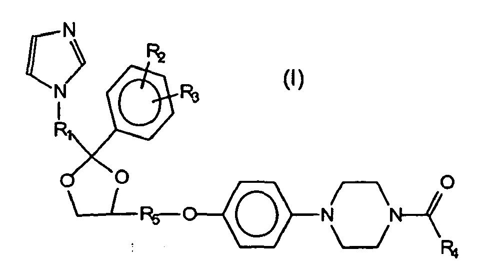 wherein R₁ and R₅ which may be the same or different represent a C₁₋₃ alkyl group, R₂ and R₃ which may be the same or different represent hydrogen, a halogen atom or a methyl group substituted by one or more halogen atoms and R₄ represents the group (CH₂)ₙCH₃ wherein n may be 0, 1 or 2 and a lipid excipient, at least 85% by weight of said lipid excipient consisting of one or more low melting point lipids wherein said low melting point lipid has a melting point less than 30°C, and 1-15% by weight of said lipid excipient consisting of one or more high melting point lipids, wherein said high melting point lipid has a melting point greater than 30°C.

2. A pharmaceutical composition as claimed in claim 1 wherein said one or more high melting point lipids is present in a quantity sufficient to provide a liquid and a solid phase within said composition after heating to 35°C for 18 hours.

3. A composition as claimed in claim 1 wherein 2-10% by weight of said lipid excipient consists of one or more high melting point lipids.

4. A composition as claimed in claim 1 wherein 2-5% by weight of said lipid excipient consists of one or more high melting point lipids.

5. A composition as claimed in any preceding claim wherein said low melting point lipid(s) are esters of fatty acids having more than 6 carbon atoms.

6. A composition as claimed in claim 5 wherein said low melting point lipid(s) are esters of fatty acids having between 16 and 22 carbon atoms.

7. A composition as claimed in claim 5 or claim 6 wherein said low melting point lipid(s) are esters of unsaturated fatty acids.

8. A composition as claimed in any one of claims 5 to 7 wherein said low melting point lipid(s) are monoglycerides.

9. A composition as claimed in any one of claims 5 to 8 wherein said low melting point lipid(s) are glycerol monooleate and/or glycerol monolinoleate.

10. A composition as claimed in any one of claims 5 to 9 wherein said low melting point lipid is glycerol monooleate.

11. A composition as claimed in any preceding claim wherein said high melting point lipid(s) are esters of fatty acids having more than 6 carbon atoms.

12. A composition as claimed in claim 9 wherein said high melting point lipid(s) are esters of fatty acids having more than 12 carbon atoms.

13. A composition as claimed in claim 12 wherein said high melting point lipid(s) are esters of fatty acids having between 16 and 22 carbon atoms.

14. A composition as claimed in any of claims 11 to 13 wherein said high melting point lipid(s) are esters of saturated fatty acids.

15. A composition as claimed in claim 14 wherein said saturated fatty acids are selected from the group comprising lauric acid, myristic acid, palmitic acid, stearic acid, arachidic acid, behenic acid, lignoceric acid and nervonic acid.

16. A composition as claimed in any one of claims 11 to 13 wherein said high melting point lipid(s) are glycerides.

17. A composition as claimed in any one of claims 11 to 16 wherein said high melting point lipid(s) are monoglycerides.

18. A composition as claimed in any one of claims 11 to 17 wherein said high melting point lipid(s) are selected from the group comprising glycerol monopalmitate, glycerol monostearate and glycerol monobehenate.

19. A composition as claimed in any preceding claim which comprises 5-30% by weight of ketoconazole in the form of a racemic or other mixture of the two cis isomers or one of the cis isomers.

20. A method of preparing a composition as claimed in any preceding claim which comprises melting and mixing the excipients and simultaneously or subsequently adding the ketoconazole or the compound of formula I.

21. A composition as claimed in any preceding claim for use in the prevention or treatment of metabolic syndrome or diabetes mellitus type II or symptoms or complications thereof.

## Patentansprüche

1. Pharmazeutische Zusammensetzung umfassend Ketoconazol oder eine Verbindung der Formel I 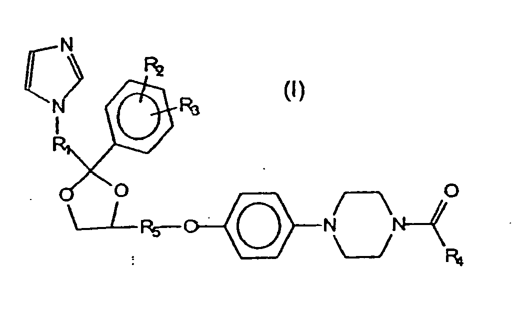 wobei R₁ und R₅, welche gleich oder verschieden sein können, eine C₁₋₃ Alkylgruppe darstellen, R₂ und R₃, welche gleich oder verschieden sein können Wasserstoff, ein Halogenatom oder eine Methylgruppe, welche mit einem oder mehreren Halogenatomen substituiert ist, darstellen und R₄ die Gruppe (CH₂)ₙCH₃ darstellt, wobei n 0, 1 oder 2 sein kann, und ein Lipid-Hilfsmittel, wobei wenigstens 85 Gewichtsprozent des Lipid-Hilfsmittels aus einem oder mehreren Lipiden mit niedrigem Schmelzpunkt bestehen, wobei das Lipid mit niedrigem Schmelzpunkt einen Schmelzpunkt von weniger als 30 °C aufweist, und 1-15 Gewichtsprozent des Lipid-Hilfsmittels aus einem oder mehreren Lipiden mit hohem Schmelzpunkt bestehen, wobei das Lipid mit hohem Schmelzpunkt einen Schmelzpunkt größer als 30 °C aufweist.

2. Pharmazeutische Zusammensetzung nach Anspruch 1, wobei das eine oder die mehreren Lipide mit hohem Schmelzpunkt in einer Menge vorliegen, welche ausreichend ist, eine flüssige und eine feste Phase in der Zusammensetzung nach Erwärmen auf 35 °C für 18 Stunden bereitzustellen.

3. Zusammensetzung nach Anspruch 1, wobei 2-10 Gewichtsprozent des Lipid-Hilfsmittels aus einem oder mehreren Lipiden mit hohem Schmelzpunkt bestehen.

4. Zusammensetzung nach Anspruch 1, wobei 2-5 Gewichtsprozent des Lipid-Hilfsmittels aus einem oder mehreren Lipiden mit hohem Schmelzpunkt bestehen.

5. Zusammensetzung nach einem der vorausgehenden Ansprüche, wobei das/die Lipid(e) mit niedrigem Schmelzpunkt (ein) Ester von Fettsäuren mit mehr als 6 Kohlenstoffatomen ist/sind.

6. Zusammensetzung nach Anspruch 5, wobei das/die Lipid(e) mit niedrigem Schmelzpunkt (ein) Ester von Fettsäuren, welche zwischen 16 und 22 Kohlenstoffatomen aufweisen, ist/sind.

7. Zusammensetzung nach einem der Ansprüche 5 oder 6, wobei das/die Lipid(e) mit niedrigem Schmelzpunkt (ein) Ester von ungesättigten Fettsäuren ist/sind.

8. Zusammensetzung nach einem der Ansprüche 5 bis 7, wobei das/die Lipid(e) mit niedrigem Schmelzpunkt (ein) Monoglycerid(e) ist/sind.

9. Zusammensetzung nach einem der Ansprüche 5 bis 8, wobei das/die Lipid(e) mit niedrigem Schmelzpunkt Glycerinmonooleat und/oder Glycerinmonolinoleat ist/sind.

10. Zusammensetzung nach einem der Ansprüche 5 bis 9, wobei das Lipid mit niedrigem Schmelzpunkt Glycerinmonooleat ist.

11. Zusammensetzung nach einem der vorausgehenden Ansprüche, wobei das/die Lipid(e) mit hohem Schmelzpunkt (ein) Ester von Fettsäuren mit mehr als 6 Kohlenstoffatomen ist/sind.

12. Zusammensetzung nach Anspruch 9, wobei das/die Lipid(e) mit hohem Schmelzpunkt (ein) Ester von Fettsäuren mit mehr als 12 Kohlenstoffatomen ist/sind.

13. Zusammensetzung nach Anspruch 12, wobei das/die Lipid(e) mit hohem Schmelzpunkt (ein) Ester von Fettsäuren, welche zwischen 16 und 22 Kohlenstoffatomen aufweisen, ist/sind.

14. Zusammensetzung nach einem der Ansprüche 11 bis 13, wobei das/die Lipid(e) mit hohem Schmelzpunkt (ein) Ester von gesättigten Fettsäuren ist/sind.

15. Zusammensetzung nach Anspruch 14, wobei die gesättigten Fettsäuren ausgewählt sind aus der Gruppe umfassend Laurinsäure, Myristinsäure, Palmitinsäure, Stearinsäure, Arachinsäure, Behensäure, Lignocerinsäure und Nervonsäure.

16. Zusammensetzung nach einem der Ansprüche 11 bis 13, wobei das/die Lipid(e) mit hohem Schmelzpunkt Glycerid(e) ist/sind.

17. Zusammensetzung nach einem der Ansprüche 11 bis 16, wobei das/die Lipid(e) mit hohem Schmelzpunkt Monoglycerid(e) ist/sind.

18. Zusammensetzung nach einem der Ansprüche 11 bis 17, wobei das/die Lipid(e) mit hohem Schmelzpunkt ausgewählt ist/sind aus der Gruppe umfassend Glycerinmonopolamitat, Glycerinmonostearat und Glycerinmonobehenat.

19. Zusammensetzung nach einem der vorausgehenden Ansprüche, welche 5-30 Gewichtsprozent Ketoconazol in Form eines racemischen oder eines anderen Gemischs aus den zwei cis-Isomeren oder von einem der cis-Isomere umfasst.

20. Verfahren zur Herstellung einer Zusammensetzung nach einem der vorausgehenden Ansprüche, welches umfasst Schmelzen und Mischen der Hilfsmittel und gleichzeitiges oder anschließendes Zugeben des Ketoconazols oder der Verbindung der Formel 1.

21. Zusammensetzung nach einem der vorausgehenden Ansprüche zur Verwendung bei der Vorbeugung oder Behandlung von Metabolischem Syndrom oder Diabetes mellitus Typ II oder Symptomen oder Komplikationen davon.

## Revendications

1. Composition pharmaceutique comprenant du cétoconazole ou un composé de formule I 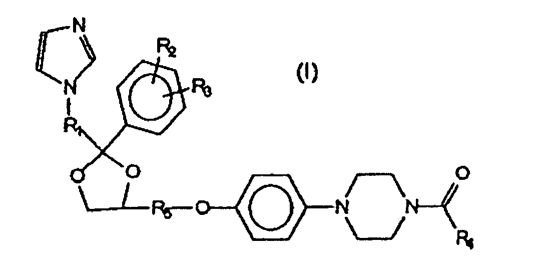 dans laquelle R₁ et R₅, qui peuvent être identiques ou différents, représentent un groupe alcoyle ayant de 1 à 3 atomes de carbone, R₂ et R₃, qui peuvent être identiques ou différents, représentent l'hydrogène, un atome d'halogène ou un groupe méthyle substitué par un ou par plusieurs atomes d'halogène, et R₄ représente le groupe (CH₂)ₙCH₃, n pouvant être 0, 1 ou 2 et un excipient lipidique, au moins 85 % en poids de l'excipient lipidique consistant en un ou en plusieurs lipides à bas point de fusion, le lipide à bas point de fusion ayant un point de fusion inférieur à 30°C, et de 1 à 15 % en poids de l'excipient lipide consistant en un ou en plusieurs lipides à haut point de fusion, le lipide à haut point de fusion ayant un point de fusion supérieur à 30°C.

2. Composition pharmaceutique suivant la revendication 1, dans laquelle le un ou plusieurs lipides à haut point de fusion est présent en une quantité suffisante pour donner une phase liquide et une phase solide au sein de la composition après chauffage à 35°C pendant 18 heures.

3. Composition suivant la revendication 1, dans laquelle de 2 à 10 % en poids de l'excipient lipidique consistent en un ou en plusieurs lipides à haut point de fusion.

4. Composition suivant la revendication 1, dans laquelle de 2 à 5 % en poids de l'excipient lipidique consistent en un ou en plusieurs lipides à haut point de fusion.

5. Composition suivant l'une quelconque des revendications précédentes, dans laquelle le ou les lipides à bas point de fusion sont des esters d'acides gras ayant plus de 6 atomes de carbone.

6. Composition suivant la revendication 5, dans laquelle le ou les lipides à bas point de fusion sont des esters d'acides gras ayant de 16 à 22 atomes de carbone.

7. Composition suivant la revendication 5 ou la revendication 6, dans laquelle le ou les lipides à bas point de fusion sont des esters d'acides gras insaturés.

8. Composition suivant l'une quelconque des revendications 5 à 7, dans laquelle le ou les lipides à bas point de fusion sont des monoglycérides.

9. Composition suivant l'une quelconque des revendications 5 à 8, dans laquelle le ou les lipides à bas point de fusion sont du monooléate de glycérol et/ou du monolinoléate de glycérol.

10. Composition suivant l'une quelconque des revendications 5 à 9, dans laquelle le lipide à bas point de fusion est du monooléate de glycérol.

11. Composition suivant l'une quelconque des revendications précédentes, dans laquelle le ou les lipides à haut point de fusion sont des esters d'acides gras ayant plus de 6 atomes de carbone.

12. Composition suivant la revendication 9, dans laquelle le ou les lipides à haut point de fusion sont des esters d'acides gras ayant plus de 12 atomes de carbone.

13. Composition suivant la revendication 12, dans laquelle le ou les lipides à haut point de fusion sont des esters d'acides gras ayant entre 16 et 22 atomes de carbone.

14. Composition suivant l'une quelconque des revendications 11 à 13, dans laquelle le ou les lipides à haut point de fusion sont des esters d'acides gras saturés.

15. Composition suivant la revendication 14, dans laquelle les acides gras saturés sont choisis dans le groupe comprenant l'acide laurique, l'acide myristique, l'acide palmitique, l'acide stéarique, l'acide arachidique, l'acide béhénique, l'acide lignocérique et l'acide nervonique.

16. Composition suivant l'une quelconque des revendications 11 à 13, dans laquelle le ou les lipides à haut point de fusion sont des glycérides.

17. Composition suivant l'une quelconque des revendications 11 à 16, dans laquelle le ou les lipides à haut point de fusion sont des monoglycérides.

18. Composition suivant l'une quelconque des revendications 11 à 17, dans laquelle le ou les lipides à haut point de fusion sont choisis dans le groupe comprenant le monopalmitate de glycérol, le monostéarate de glycérol et le monobéhénate de glycérol.

19. Composition suivant l'une quelconque des revendications précédentes, qui comprend de 5 à 30 % en poids de cétoconazole sous la forme d'un racémique ou d'autres mélanges des deux isomères cis ou l'un des isomères cis.

20. Procédé de préparation d'une composition telle que revendiquée à l'une quelconque des revendications précédentes, dans lequel on fait fondre et on mélange les excipients et, en même temps ou ensuite, on ajoute le cétoconazole ou le composé de formule I.

21. Composition suivant l'une quelconque des revendications précédentes, à utiliser dans la prévention ou dans le traitement du syndrome métabolique ou du diabète sucré de type II ou leurs symptômes ou leurs complications.
